# EUROPEAN PATENT APPLICATION

(11) **EP 1 098 002 A1**
(43) Date of publication of application: **09.05.2001**
(21) Application number: 99919278.4
(22) Date of filing: 11.05.1999
(51) Int. Cl.: C12Q 1/14

(54) **DEHYDRATED IMMEDIATE RECONSTRUCTION CULTURE MEDIUM TO IDENTIFY GROUP B STREPTOCOCCI i STREPTOCOCCUS AGALACTIAE /i BY THE DETECTION OF THEIR PIGMENT**

(71) Applicant: De La Rosa Fraile, Manuel, 18014 Granada (ES)
(72) Inventor: De La Rosa Fraile, Manuel, 18014 Granada (ES)
(74) Representative: De Pablos Riba, Julio
(86) International application number: ES9900130
(87) International publication number: WO0068417

(57) **Abstract**

The invention consists of a dehydrated culture medium for detection of *Streptococcus agalactiae* based on pigment detection which requires merely the addition of water and then shaking to make it ready for use. The culture medium when rehydrated turns into a semisolid gel that allows the growth of *S.agalactiae* inside the medium as orange or red colonies allowing its easy detection.

The new instant medium comprises a dehydrated culture medium plus a getting agent e.g. Sephadex 200. When the user adds water a room temperature and shake a gel is form immediately and the medium is ready to use. *Streptococcus agalactiae* grows in this medium as pigmented colonies allowing for easy detection. The rehydrated medium is thick enough to allow the bacteria not to sediment and the gelling process is quick enough to trap air bubbles during the shaking. These trapped air bubbles remain inside the medium during the incubation time creating interfacial environments where *S.agalactiae* grows as strongly pigmented colonies.

## Description

### BACKGROUND OF THE INVENTION

### Introduction

*Streptococcus agalactiae*, Lancefield Group B streptococcus (GBS) is an important human pathogen that causes infections mainly in newborns, pregnant women, and immunocompromised people. GBS is also an important animal pathogen. It is therefore important to get reliable and easy to use diagnostic systems to detect GBS from clinical samples. One of the easiest of these systems is to culture clinical specimens in Granada Medium (De la Rosa et al. J. Clin. Microbial. 1992;30: 1019-1021 and Spanish Patent 91-02833)

This culture medium use agar as a gelling agent and so their preparation requires heating to prepare it. In addition, this medium contain labile biological components (e.g. horse serum) which deteriorate quickly giving a shelf life of only of 1 - 2 months.

### State-of-the-art prior to this invention

The principles that rule the decay of prepared culture media are little understood, though they should be similar to those that govern the decay of complex mixtures of natural products in the presence of water (Postgate 1963, Martin 1971 Bridson 1978, Cote 1994).

In general the expiry date of prepared culture media is less than two months mainly because of: i) Decay of unstable components, ii) Chemical reaction of medium components with the oxygen in air, that leads to oxidation and formation of peroxides and superoxides, and iii) Ageing of the gelling agent. These problems are specially relevant in culture media that contain complex biological products (e.g. blood or serum) or selective agents (antibiotics).

Different approaches to tackle the problem of long term stability of culture media have been proposed, but to date no satisfactorily solution has been found. Difco (Detroit, USA, Michigan) marketed a special brand of prepared culture media in plates with an improved expiry date that used special hermetic containers (Durapak plates). Oxoid Ltd. (Basingtoke, UK) markets culture media devoid of non-stable components, with separate freeze-dried supplements (selective supplements). These supplements are reconstituted (rehydrated) immediately before use and are added to the medium base, that after sterilization or melting is allowed to cool to about 50 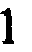 C.

### Relevant references about culture media stability

BRIDSON E.Y. 1978. Natural and synthetic culture media for bacteria pp. 91-281. In Handbook series in nutrition and food. Vol. III. Section G: Diets, Culture media and food supplements. Rechcigl Jr. M. (Ed.). CRC. Press Inc. Cleveland. Ohio
BRIDSON E.Y. 1990. The Oxoid Manual 6^{th} Ed. Oxoid Unipath Limited, Basingtoke, England
COTE R.J., GHERNA R.L. 1994. Nutrition and Media. pp 155-178. In Methods for General and Molecular Bacteriology. Gerhard OP, Murray R.G.E., Wood W.A., Krieg R. N. Ed. American Society for Microbiology Washington.
MARTIN R. J. 1971. Storage of microbial culture media. Lab. Pract. 90:653-656
POSTGATE J. R. 1963. Chemical stability of biologicaly important derivatives. J. Gen. Microbiol 30:481-484

### Relevant references about culture media for S.agalactiae detection based on observation of its pigment

FALLON J.R. 1974. The rapid recognition of Lancefield Group B hemolytic streptococci. J. Clin. Path. 27:902-905.
ISLAM A.K 1977.Rapid recognition of group B streptococci. Lancet. i:256-7
MERRIT K, JACOBS N.J. 1976. Improved medium for detecting pigment production by group B streptococci. J. Clin. Microbiol. 4:379-380.
MERRIT K, JACOBS N.J. 1978. Characterization and incidence of pigment production by human clinical group B streptococci. J. Clin. Microbiol. 8:105-107.
PERSSON K.M.S., FORSGREN A 1987. Evaluation of culture methods for isolation of group B streptococci. Diag. Microbial, Infect. Dis. 6:175-177.
ROSA M., VILLARREAL R., VEGA D., MIRANDA C., MARTINEZ-BROCAL A. 1983. Granada Medium for detection and identification of Group B Streptococci. J. Clin. Microbiol. 18:779-785.
ROSA FRAILE M. 1991. Spanish Patent 91/02833. Medio de cultivo diagnóstico de *Streptococcus agalactiae* por detección de su pigmento y que activa la formación de este pigmento par el metotrexate y/o otros inhibidores de la via del folato
ROSA M., PEREZ M., CARAZO C., PEIS J.I. 1992. New Granada Medium for detection and Identification of Group B streptococci. J. Clin. Microbiol. 30:1019-1021.
ROSA FRAILE M. 1995. Spanish Patent 95/00365. Medio de cultivo pare *Streptococcus agalactiae* que activa la formación de pigmento por amilasas y/o glucooligosacáridos [G( 1-4)G]n (n>2), adicionados como compuestos quimicos, como hidrolizados de polisacáridos o generados en el mismo medio con enzimas y polisacáridos.
ROSA-FRAILE M., SAMPEDRO A, RUIZ-BRAVO A., SANBONMATSU S., GIMENEZ-GALLEGO G. 1996. Serum and urine proteins responsible for enhanced pigment production by Group B streptococci identified as amylases. Clin. Diagn. Lab. Immunol. 3:594-596.
M. ROSA-FRAILE, A SAMPEDRO, J. VARELA, M. GARCIA-PEÑA, G. GIMENEZ-GALLEGO. 1999. Identification of a peptide from mammal albumins responsible for enhanced pigment production by group B streptococci. Clin. Diagn. Lab. Immunol.
ROSA FRAILE M. 1999. Spanish Patent 99/00443. Sistema que evita la incubación anaerobia de places de cultivo para detectar *Streptococcus agalactiae* por su pigmento forzando el crecimiento coplanario
SCHAUFUß P., LÄMMLER CH., BLOBEL H. 1985. Effects of glucose and trimethoprim on pigment production of group B streptococci. IRCS Med. Sci. 13:842

### THE INVENTION

### Summary of the invention

This invention is a practical and conceptual improvement of culture media for the diagnosis of *S.agalactiae* based on observation of its pigment which does not suffer from a limited shelf life, the main drawback of previous culture media (e.g. Granada Medium).

The present invention provides an instant and stable culture medium (e.g. Instant Granada Medium, IGM), which is a dehydrated culture medium using Sephadex 200 (Amershan Pharmacia Biotech, Sweden) (or Sephadex, alginate and/or other thickening or gelling agents) as an instant gelling agent. After being rehydrated with water or a salt solution the medium gels quickly at room temperature forming a culture medium thick enough to: **i)** Allow bacterial growth inside the solidified medium without settling, **ii)** Withstand being turned over without spilling, and **iii)** Trap small air bubbles during the shaking when rehydrating. These bubbles remain inside the medium during incubation. In addition the medium is transparent enough to allow easy observation of pigmented GBS colonies.

The small bubbles of air trapped during the gelling process create discontinuities and interfaces between the medium and the trapped air that produce special environments (Codner, R.C. 1969. Solid and solidified growth media in microbiology, p. 427-454. *In* J.R. Norris y D.W. Ribbons (eds.), Methods in Microbiology, Vol.1. Academic Press, London) that trigger pigment production in the same way as coplanar growth described in the Spanish Patent Application 9900443. The resultant culture medium is used in transparent glass or plastic tubes and the pigmented GBS colonies are observed or detected inside the transparent or translucent culture medium.

### General description of the invention

The production of the instant culture medium needs the preparation of a dry culture medium base e.g. Granada Medium (Spanish patent 91-02833 and Rosa et at J. Clin. Microbiol. 1992;30:1019-1021) prepared without agar and lyophilized afterwards. This dry medium is thoroughly mixed with enough getting agent e.g. Sephadex 200, to produce a final concentration of 5-6 % in the rehydrated medium. The proportion of gelling agent to the dry medium base should be adjusted so as to get a final dry complete medium that after rehydrating and shaking has the required thickness as described in the summary.

In addition to Sephadex 200 any other gelling agent or mixtures of gelling agents able to rehydrate quickly at room temperature rapidly giving (after shaking or mixing) a media with the required characteristics (as stated in the summary) can be used e.g. alginate, galactomannan, gum xantham etc. Gelling agents that gel when mixing with cations can also be used e.g. alginic acid, carrageenan or gelrite gellan gum (Phytagel).

The characteristics and differential features of the instant culture medium are: **i)** Instant rehydration at room temperature of a stable dry medium, **ii)** A resulting gelled medium thick enough to trap bubbles of air creating interfaces, and **iii)** Bacterial growth produced inside the medium without settling.

### EXAMPLE

This example illustrates the preparation of Instant Granada Medium (IGM) from New Granada Medium (Spanish Patent 9102833 and Rosa et al J. Clin. Microbiol. 1992; 30:1019-1021)
**1)** Basal medium.
   Weight: Suitable peptone 25 g (e.g. Difco Proteose Peptone No3) (Rosa-Fraile M. et al. 1999). Identification of a peptide from mammal albumins responsible for enhanced pigment production by group B streptococci. Clin. Diagn. Lab. Inmunol. 1999; 6:425-426); Soluble starch 10 g; Disodium phosphate (Na₂HPO₄) 8.5 g; MOPS (3-(M-Morpholino) propane sulphonic acid) hemisodium salt 11 g. Suspend in one liter distilled water. Bring gently to the boil with frequent agitation to dissolve completely. Sterilize by autoclaving for 20 minutes at 0.5 atm (do not overheat).
**2)** Supplement.
   Methotrexate (sodium salt) 0.6 g/l, glucose 250 g/l, anhydrous magnesium sulphate (MgSO₄) 20 g/l; sodium pyruvate 100 g/l; Crystal violet 20 mg/l, Colistin 500 mg/l, Metronidazole 1 g/l.
   Sterilize by filtration.
**3)** Complete medium base.
   Add 10 ml of supplement and 50 ml of horse serum to 1 liter of cooled basal medium.
**4)** Lyophilize.
**5)** Addition of the gelling agent.
   Mix with Sephadex 200. For each 100 g of lyophilized medium base add 73.3 g of Sephadex 200. Grind a to fine powder mixing carefully.
**6)** Distribute the powder in transparent tubes.
   Use plastic or glass tubes with hermetic caps (130 mg per ml of water to be added to the dehydrated medium) e.g. 520 mg of dry medium in 5 cm high x 14 mm inner diameter tubes.
**7)** Rehydration.
   Add to the tubes containing the dry medium 1 ml of sterile distilled water for each 130 mg of dry medium. Shake vigorously several times until all the powder is suspended in the water.
   If the medium is to be used to detect GBS from clinical sample swabs, it is advisable to put the swab in the tubes with the dry medium, and then add the necessary amount of water cap the tube and shake.
**8)** Sample inoculation.
   If the method described in point 7 is not used, inoculate the sample after the gelling process is completed (1-3 minutes).
**9)** Incubation.
   Tubes should be incubated at 35-37 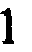 C for 18-48 h., preferably in a water bath. If a swab has been used it should be left inside the medium during incubation.
**10)** Results.
   GBS growth will be observed as orange or red spots or lumps inside the medium.

## Claims

1. An instant and extended shelf life culture medium for detection and identification of Group B Streptococci. This culture medium is characterized by using as gelling agent a substance o mixture of substances that after the addition of water gels immediately at room temperature producing a culture medium thick enough to allow Group B Streptococci to grow without settling.

2. An instant culture medium according to claim 1 characterized by using Sephadex 200 as gelling agent.

3. An instant culture medium according to claims 1 and 2 characterized by using New Granada Medium as medium base for *Streptococcus agalactiae* pigment detection.

4. An instant culture medium according to claim 1 characterized by its ability to trap air bubbles during the shaking in the rehydration process which remain in the medium during incubation creating interfacial surfaces that trigger *Streptococcus agalactiae* pigment production.
